# EUROPEAN PATENT SPECIFICATION

(11) **EP 1 569 602 B1**
(45) Date of publication and mention of the grant of the patent: **28.02.2007**
(21) Application number: 03785736.4
(22) Date of filing: 26.11.2003
(51) Int. Cl.: A61Q 5/00, A61K 8/73, A61K 8/362

(54) **HAIR TREATMENT COMPOSITIONS**
HAARBEHANDLUNGSMITTEL
COMPOSITIONS CAPILLAIRES TRAITANTES

(30) Priority: 13.12.2002 EP 02258604
(43) Date of publication of application: 07.09.2005
(73) Proprietor: UNILEVER PLC, London EC4P 4BQ (GB); UNILEVER N.V., 3013 AL Rotterdam (NL)
(72) Inventor: CORNWELL, Paul A., Unilever R & D Port Sunlight, Wirral, Merseyside CH63 3JW (GB); HULL, Peter, James, Knutsford, Cheshire WA16 9AH (GB); SKINNER, Richard, Unilever R & D Port Sunlight, Wirral, Merseyside CH63 3JW (GB); DEVINE, Karen Maria, Unilever R & D Port Sunlight, Wirral, Merseyside CH63 3JW (GB)
(74) Representative: Tansley, Sally Elizabeth
(86) International application number: PCT/EP2003/013701
(87) International publication number: WO 2004/054526

(56) References cited:
- EP-A- 0 383 467
- WO-A-00/40213
- WO-A-01/01948
- DE-B- 1 220 969
- US-B1- 6 348 200
- PATENT ABSTRACTS OF JAPAN vol. 1998, no. 03, 27 February 1998 (1998-02-27) & JP 09 291011 A (KOSE CORP), 11 November 1997 (1997-11-11)
- PATENT ABSTRACTS OF JAPAN vol. 011, no. 300 (C-449), 29 September 1987 (1987-09-29) & JP 62 091236 A (NIPPON OIL & FATS CO LTD;OTHERS: 01), 25 April 1987 (1987-04-25)
- DATABASE WPI Section Ch, Week 198649 Derwent Publications Ltd., London, GB; Class D21, AN 1986-322321 XP002241480 & JP 61 238894 A (SUNSTAR KK), 24 October 1986 (1986-10-24)
- PATENT ABSTRACTS OF JAPAN vol. 1999, no. 01, 29 January 1999 (1999-01-29) & JP 10 279449 A (NICHIDEN KAGAKU KK), 20 October 1998 (1998-10-20)

## Description

### FIELD OF THE INVENTION

The invention relates to a method of treating hairs. More particularly the invention relates to a method of hair treatment comprising specific combinations of active materials. These methods are particularly suitable for application to hair for repair and restoration of damaged hair.

### BACKGROUND AND PRIOR ART

Hair can suffer damage from a number of sources such as; exposure to UV and chlorine; chemical influences such as bleaching, perming, overly frequent washing with harsh surfactant-based cleansing shampoo compositions; and mechanical influences such as prolonged use of heated styling appliances.

Damage to the hair typically manifests itself in cuticle and protein loss from the hair fibre, hair fibre brittleness and breakage and frayed or split ends.

In addition consumers require their hair to be manageable; that is that the hair when styled will retain that style for a long period of time and in a range of environmental detrimental conditions such as high humidity.

WO 97/14401 (Kao Corporation)describes a wide range of organic acids in skin and hair care compositions.

The use of sugars and plant extracts are disclosed in WO 01/68040 (L'Oreal) to protect keratinous tissue. Trehalose has been used to improve the elasticity of skin and/or prevent ageing as disclosed in WO 01/01948.

The present invention has now found that compositions comprising certain specific combinations of sugars and acids are effective for repairing and preventing the principal symptoms of damaged hair, these combinations also have the further advantage that they help to increase the manageability of the hair.

### DESCRIPTION OF THE INVENTION

In a first aspect, the present invention provides a method for treating hair comprising the step of applying to the hair a composition comprising i) from 0.05 wt.% to 49 wt.% of a disaccharide; and ii) a di- acid.

A further aspect of the invention is the use of the above composition for smoothing hair, aligning hair and preventing damage to the hair.

WO 01/01948 discloses skin care compositions comprising trehalose for moistering and protecting elastine fibers.

### Detailed Description

### The disaccharide

The present invention comprises as an essential element of the invention a disaccharide, preferably the disaccharide comprises of pentose or hexose sugars, more preferably the disaccharide comprises of two hexose units.

Disaccharides can be either reducing or non-reducing sugars. Non-reducing sugars are preferred.

The D(+) form of the sugars are preferred. Particularly preferred are trehalose and cellobiose or mixtures thereof. Trehalose is the most preferred disaccharide.

The level of disaccharides present in the total formulation is from 0.05 wt% to 49 wt%, more preferably from 0.2wt% to 3 wt%, most preferably from 0.5wt% to 2wt%.

### Di-Acids

Di-acids are present in the compositions of the present invention, particularly suitable are di-acids having the formula:

HOOC-(CH₂)ₙ-COOH

where n is an integer from 2 to 8, more preferably where n equal to 2 or 4 (succinic acid and adipic acid respectively).

Organic acids are best used at levels in the total formulation from 0.01 wt% to 5wt%, more preferably at levels from 0.1wt% to 2wt%.

The weight ratio of di-acid to disaccharide is 1:10 to 20:1, more preferably 1:5 to 5:1.

In addition, acids are best used at di-acid:disaccharide molar ratios of between 0.1:1 and 10:1, preferably between 0.1:1 and 2:1.

The pH of the formulations of the invention are in the range from pH 3 to pH 6, more preferably used at pH 3-5.

### Guanidinium Salt

Optionally a guanidinium salt is present. For example, guanidinium carbonate, guanidinium sulphate and guanidinium phosphate. Particularly preferred is guanidinium carbonate. Guanidinium salts are best used at levels from 0.01wt%w to 5wt.% of the total formulation, more preferably at 0.1wt%w to 2wt%.

The preferred weight ratio of guanidinium salt to disaccharide is from 1:10 to 4:1, more preferably 2:1 to 1:2.

In addition the preferred guanidinium salt:disaccharide molar ratios are from 0.1:1 to 10:1, more preferably from 0.1:1 to 2:1.

### Product Form

The final product form of hair treatment compositions according to the invention may suitably be, for example, shampoos, conditioners, sprays, mousses, gels, waxes or lotions. Particularly preferred product forms are shampoos, post-wash conditioners (leave-in and rinse-off) and hair treatment products such as hair essences.

Shampoo compositions preferably comprise one or more cleansing surfactants which are cosmetically acceptable and suitable for topical application to the hair. Further surfactants may be present as emulsifiers.

Suitable cleansing surfactants, are selected from anionic, amphoteric and zwitterionic surfactants, and mixtures thereof. The cleansing surfactant may be the same surfactant as the emulsifier, or may be different.

### Anionic Cleansing Surfactant

Shampoo compositions according to the invention will typically comprise one or more anionic cleansing surfactants which are cosmetically acceptable and suitable for topical application to the hair.

Examples of suitable anionic cleansing surfactants are the alkyl sulphates, alkyl ether sulphates, alkaryl sulphonates, alkanoyl isethionates, alkyl succinates, alkyl sulphosuccinates, N-alkyl sarcosinates, alkyl phosphates, alkyl ether phosphates, alkyl ether carboxylates, and alphaolefin sulphonates, especially their sodium, magnesium, ammonium and mono-, di- and triethanolamine salts. The alkyl and acyl groups generally contain from 8 to 18 carbon atoms and may be unsaturated. The alkyl ether sulphates, alkyl ether phosphates and alkyl ether carboxylates may contain from 1 to 10 ethylene oxide or propylene oxide units per molecule.

Typical anionic cleansing surfactants for use in shampoo compositions of the invention include sodium oleyl sulpho succinate, ammonium lauryl sulphosuccinate, ammonium lauryl sulphate, sodium cocoyl isethionate, sodium lauryl isethionate and sodium N-lauryl sarcosinate. The most preferred anionic surfactants are sodium lauryl sulphate, sodium lauryl ether sulphate(n)EO, (where n ranges from 1 to 3), ammonium lauryl sulphate and ammonium lauryl ether sulphate(n)EO, (where n ranges from 1 to 3).

The total amount of anionic cleansing surfactant in shampoo compositions of the invention is generally from 5 to 30, preferably from 6 to 20, more preferably from 8 to 16 wt%.

### Co-surfactant

The shampoo composition can optionally include co-surfactants, preferably an amphoteric or zwitterionic surfactant, which can be included in an amount ranging from 0 to about 8, preferably from 1 to 4 wt%.

Examples of amphoteric and zwitterionic surfactants include, alkyl betaines, alkyl amidopropyl betaines, alkyl sulphobetaines (sultaines), alkyl glycinates, alkyl carboxyglycinates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine and preferably lauryl betaine, cocamidopropyl betaine and sodium cocamphopropionate.

Another preferred co-surfactant is a nonionic surfactant, which can be included in an amount ranging from 0 to 8, preferably from 2 to 5 wt%.

For example, representative nonionic surfactants that can be included in shampoo compositions of the invention include condensation products of aliphatic (C₈ - C₁₈) primary or secondary linear or branched chain alcohols or phenols with alkylene oxides, usually ethylene oxide and generally having from 6 to 30 ethylene oxide groups.

Further nonionic surfactants which can be included in shampoo compositions of the invention are the alkyl polyglycosides (APGs). Typically, the APG is one which comprises an alkyl group connected (optionally via a bridging group) to a block of one or more glycosyl groups. Preferred APGs are defined by the following formula:

RO - (G)ₙ

wherein R is a branched or straight chain C₅ to C₂₀ alkyl or alkenyl group, G is a saccharide group and n is from 1 to 10.

Other sugar-derived nonionic surfactants which can be included in shampoo compositions of the invention include the C₁₀-C₁₈ N-alkyl (C₁-C₆) polyhydroxy fatty acid amides, such as the C₁₂-C₁₈ N-methyl glucamides, as described for example in WO 92 06154 and US 5 194 639, and the N-alkoxy polyhydroxy fatty acid amides, such as C₁₀-C₁₈ N-(3-methoxypropyl) glucamide.

The shampoo composition can also optionally include one or more cationic co-surfactants included in an amount ranging from 0.01 to 10, more preferably from 0.05 to 5, most preferably from 0.05 to 2 wt%. Useful cationic surfactants are described hereinbelow in relation to conditioner compositions.

The total amount of surfactant (including any co-surfactant, and/or any emulsifier) in shampoo compositions of the invention is generally from 5 to 50, preferably from 5 to 30, more preferably from 10 to 25 wt%.

### Cationic Polymer

A cationic polymer may be present. The cationic polymer may be a homopolymer or be formed from two or more types of monomers. The molecular weight of the polymer will generally be between 5 000 and 10 000 000, typically at least 10 000 and preferably in the range 100 000 to about 2 000 000. The polymers will have cationic nitrogen containing groups such as quaternary ammonium or protonated amino groups, or a mixture thereof.

Suitable cationic nitrogen polymers are described in the CTFA Cosmetic Ingredient Directory, 3rd edition

The cationic conditioning polymers can comprise mixtures of monomer units derived from amine- and/or quaternary ammonium-substituted monomer and/or compatible spacer monomers.

Suitable cationic conditioning polymers include, copolymers of 1-vinyl-2-pyrrolidine and 1-vinyl-3-methylimidazolium salt (CTFA name Polyquaternium-16); copolymers of 1-vinyl-2-pyrrolidine and dimethylaminoethyl methacrylate, (CTFA name Polyquaternium-11); cationic diallyl quaternary ammonium-containing polymers in particular (CTFA Polyquaternium 6 and Polyquaternium 7, mineral acid salts of amino-alkyl esters of homo-and copolymers of unsaturated carboxylic acids as described in U.S. Patent 4,009,256; cationic polyacrylamides(as described in WO95/22311).

Cationic polysaccharide polymers suitable for use in compositions of the invention include those with an anhydroglucose residual group, such as a starch or cellulose. Cationic cellulose is available from Amerchol Corp. (Edison, NJ, USA) in their Polymer JR (trade mark) and LR (trade mark) series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from Amerchol Corp. (Edison, NJ, USA) under the tradename Polymer LM-200.

Other suitable cationic polysaccharide polymers include quaternary nitrogen-containing cellulose ethers (e.g. as described in U.S. Patent 3,962,418), and copolymers of etherified cellulose and starch (e.g. as described in U.S. Patent 3,958,581).

A particularly suitable type of cationic polysaccharide polymer that can be used is a cationic guar gum derivative, such as guar hydroxypropyltrimonium chloride (commercially available from Rhone-Poulenc in their JAGUAR trademark series). Particularly preferred cationic polymers are JAGUAR C13S, JAGUAR C14, JAGUAR C15, JAGUAR C17 and JAGUAR C16 Jaguar CHT and JAGUAR C162.

The cationic conditioning polymer will generally be present in compositions of the invention at levels of from 0.01 to 5, preferably from 0.05 to 1, more preferably from 0.08 to 0.5 wt%.

### Conditioning Surfactant

Conditioner compositions usually comprise one or more conditioning surfactants which are cosmetically acceptable and suitable for topical application to the hair.

Suitable conditioning surfactants are selected from cationic surfactants, used singly or in admixture.

Cationic surfactants useful in compositions of the invention contain amino or quaternary ammonium hydrophilic moieties which are positively charged when dissolved in the aqueous composition of the present invention.

Examples of suitable cationic surfactants are those corresponding to the general formula:

[N(R₁)(R₂)(R₃)(R₄)]⁺ (X)⁻

in which R₁, R₂, R₃, and R₄ are independently selected from (a) an aliphatic group of from 1 to 22 carbon atoms, or (b) an aromatic, alkoxy, polyoxyalkylene, alkylamido, hydroxyalkyl, aryl or alkylaryl group having up to 22 carbon atoms; and X is a salt-forming anion such as those selected from halogen, (e.g. chloride, bromide), acetate, citrate, lactate, glycolate, phosphate nitrate, sulphate, and alkylsulphate radicals.

The aliphatic groups can contain, in addition to carbon and hydrogen atoms, ether linkages, and other groups such as amino groups. The longer chain aliphatic groups, e.g., those of about 12 carbons, or higher, can be saturated or unsaturated.

The most preferred cationic surfactants for conditioner compositions of the present invention are monoalkyl quaternary ammonium compounds in which the alkyl chain length is C16 to C22.

Examples of suitable cationic surfactants include quaternary ammonium compounds, particularly trimethyl quaternary compounds.

Preferred quaternary ammonium compounds include cetyltrimethylammonium chloride, behenyltrimethylammonium chloride (BTAC), cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, cocotrimethylammonium chloride, PEG-2 oleylammonium chloride and salts of these where the chloride is replaced by halogen, (e.g. , bromide), acetate, citrate, lactate, glycolate, phosphate nitrate, sulphate, or alkylsulphate. Further suitable cationic surfactants include those materials having the CTFA designations Quaternium-5, Quaternium-31 and Quaternium-18. Mixtures of any of the foregoing materials may also be suitable. A particularly useful cationic surfactant for use in hair conditioners of the invention is cetyltrimethylammonium chloride, available commercially, for example as GENAMIN CTAC, ex Hoechst Celanese.

Salts of primary, secondary, and tertiary fatty amines are also suitable cationic surfactants. The alkyl groups of such amines preferably have from 12 to 22 carbon atoms, and can be substituted or unsubstituted.

Particularly useful are amido substituted tertiary fatty amines, in particular tertiary amines having one C₁₂ to C₂₂ alkyl or lakenyl chain. Such amines, useful herein, include stearamidopropyIdimethylamine, stearamidopropyidiethylamine, stearamidoethyldiethylamine, stearamidoethyldimethylamine, palmitamidopropyld imethylamine, palmitamidopropyldiethylamine, palmitamidoethyldiethylamine, palmitamidoethyldimethylamine, behenamidopropyldimethylamine, behenamidopropyldiethylamine, behenamidoethyldiethylamine, behenamidoethyldimethylamine, arachidamidopropyldimethylamine, arachid amidopropyldiethylamine, arachidamidoethyldiethylamine, arachidamidoethyldimethylamine, diethylaminoethylstearamide. Also useful are dimethylstearamine, dimethylsoyamine, soyamine, myristylamine, tridecylamine, ethylstearylamine, N-tallowpropane diamine, ethoxylated (with 5 moles of ethylene oxide) stearylamine, dihydroxyethylstearylamine, and arachidyl behenylamine.

These amines are typically used in combination with an acid to provide the cationic species. The preferred acid useful herein includes L- glutamic acid, lactic acid, hydrochloric acid, malic acid, succinic acid, acetic acid, fumaric acid, tartaric acid, citric acid, L-glutamic hydrochloride, and mixtures thereof; more preferably L-glutamic acid, lactic acid, citric acid. Cationic amine surfactants included among those useful in the present invention are disclosed in U.S. Patent 4,275,055 to Nachtigal, et al., issued June 23, 1981.

The molar ratio of protonatable amines to H⁺ from the acid is preferably from about 1:0.3 to 1:1.2, and more preferably from about 1:0.5 to about 1:1.1.

In the conditioners of the invention, the level of cationic surfactant is preferably from 0.01 to 10, more preferably 0.05 to 5, most preferably 0.1 to 2 wt% of the total composition.

The cationic surfactants detailed in this section are also suitable for use in the aspect of the invention wherein a cationic surfactant is intimately mixed with the thermotropic mesogenic material and with oily conditioning material prior to the incorporation of the conditioning material into the final hair conditioning composition

### Fatty Materials

Conditioner compositions of the invention preferably additionally comprise fatty materials. The combined use of fatty materials and cationic surfactants in conditioning compositions is believed to be especially advantageous, because this leads to the formation of a structured phase, in which the cationic surfactant is dispersed.

By "fatty material" is meant a fatty alcohol, an alkoxylated fatty alcohol, a fatty acid or a mixture thereof.

Preferably, the alkyl chain of the fatty material is fully saturated.

Representative fatty materials comprise from 8 to 22 carbon atoms, more preferably 16 to 22. Examples of suitable fatty alcohols include cetyl alcohol, stearyl alcohol and mixtures thereof. The use of these materials is also advantageous in that they contribute to the overall conditioning properties of compositions of the invention.

Alkoxylated, (e.g. ethoxylated or propoxylated) fatty alcohols having from about 12 to about 18 carbon atoms in the alkyl chain can be used in place of, or in addition to, the fatty alcohols themselves. Suitable examples include ethylene glycol cetyl ether, polyoxyethylene (2) stearyl ether, polyoxyethylene (4) cetyl ether, and mixtures thereof.

The level of fatty alcohol material in conditioners of the invention is suitably from 0.01 to 15, preferably from 0.1 to 10, and more preferably from 0.1 to 5 wt%. The weight ratio of cationic surfactant to fatty alcohol is suitably from 10:1 to 1:10, preferably from 4:1 to 1:8, optimally from 1:1 to 1:7, for example 1:3.

### Suspending Agents

In a preferred embodiment, the hair treatment composition, especially if it is a shampoo composition, further comprises from 0.1 to 5-wt% of a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

### Conditioning Agents

### Silicone Conditioning Agents

The compositions of the invention can contain emulsified droplets of a silicone conditioning agent, for enhancing conditioning performance.

Suitable silicones include polydiorganosiloxanes, in particular polydimethylsiloxanes that have the CTFA designation dimethicone. Also suitable for use compositions of the invention (particularly shampoos and conditioners) are polydimethyl siloxanes having hydroxyl end groups, which have the CTFA designation dimethiconol. Also suitable for use in compositions of the invention are silicone gums having a slight degree of cross-linking, as are described for example in WO 96/31188.

The viscosity of the emulsified silicone itself (not the emulsion or the final hair conditioning composition) is typically at least 10,000 cst at 25 °C the viscosity of the silicone itself is preferably at least 60,000 cst, most preferably at least 500,000 cst, ideally at least 1,000,000 cst. Preferably the viscosity does not exceed 10⁹ cst for ease of formulation.

Emulsified silicones for use in the shampoo compositions of the invention will typically have an average silicone droplet size in the composition of less than 30, preferably less than 20, more preferably less than 10 µm, ideally from 0.01 to 1 µm. Silicone emulsions having an average silicone droplet size of ≤ 0.15 µm are generally termed microemulsions.

Examples of suitable pre-formed emulsions include emulsions DC2-1766, DC2-1784, DC-1785 DC-1786 and microemulsions DC2-1865 and DC2-1870, all available from Dow Corning. These are all emulsions/microemulsions of dimethiconol. Cross-linked silicone gums are also available in a pre-emulsified form, which is advantageous for ease of formulation. A preferred example is the material available from Dow Corning as DC X2-1787, which is an emulsion of cross-linked dimethiconol gum. A further preferred example is the material available from Dow Corning as DC X2-1391, which is a microemulsion of cross-linked dimethiconol gum.

A further preferred class of silicones for inclusion in shampoos and conditioners of the invention are amino functional silicones. By "amino functional silicone" is meant a silicone containing at least one primary, secondary or tertiary amine group, or a quaternary ammonium group. Examples of suitable amino functional silicones include: polysiloxanes having the CTFA designation "amodimethicone", Specific examples of amino functional silicones suitable for use in the invention are the aminosilicone oils DC2-8220, DC2-8166, DC2-8466, and DC2-8950-114 (all ex Dow Corning), and GE 1149-75, (ex General Electric Silicones).
Suitable quaternary silicone polymers are described in EP-A-0 530 974. A preferred quaternary silicone polymer is K3474, ex Goldschmidt.

Also suitable are emulsions of amino functional silicone oils with non-ionic and/or cationic surfactant.
Pre-formed emulsions of amino functional silicone are also available from suppliers of silicone oils such as Dow Corning and General Electric. Specific examples include DC929 Cationic Emulsion, DC939 Cationic Emulsion, and the non-ionic emulsions DC2-7224, DC2-8467, DC2-8177 and DC2-8154 (all ex Dow Corning).

With some shampoos it is particularly preferred to use a combination of amino and non amino functional silicones

The total amount of silicone is preferably from 0.01 to 10 %wt of the total composition more preferably from 0.3 to 5; most preferably 0.5 to 3-wt% is a suitable level.

### (ii) Non-silicone Oily Conditioning Components

Compositions according to the present invention may also comprise a dispersed, non-volatile, water-insoluble oily conditioning agent.

By "insoluble" is meant that the material is not soluble in water (distilled or equivalent) at a concentration of 0.1% (w/w), at 25°C.

Suitable oily or fatty materials are selected from hydrocarbon oils, fatty esters and mixtures thereof. Straight chain hydrocarbon oils will preferably contain from about 12 to about 30 carbon atoms. Also suitable are polymeric hydrocarbons of alkenyl monomers, such as C₂-C₆ alkenyl monomers.

Specific examples of suitable hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, and mixtures thereof. Branched-chain isomers of these compounds, as well as of higher chain length hydrocarbons, can also be used.

Suitable fatty esters are characterised by having at least 10 carbon atoms, and include esters with hydrocarbyl chains derived from fatty acids or alcohols, Monocarboxylic acid esters include esters of alcohols and/or acids of the formula R'COOR in which R' and R independently denote alkyl or alkenyl radicals and the sum of carbon atoms in R' and R is at least 10, preferably at least 20. Di- and trialkyl and alkenyl esters of carboxylic acids can also be used.

Particularly preferred fatty esters are mono-, di- and triglycerides, more specifically the mono-, di-, and triesters of glycerol and long chain carboxylic acids such as C₁-C₂₂ carboxylic acids. Preferred materials include cocoa butter, palm stearin, sunflower oil, soyabean oil and coconut oil.

The oily or fatty material is suitably present at a level of from 0.05 to 10, preferably from 0.2 to 5, more preferably from about 0.5 to 3-wt%.

In hair treatment compositions containing a conditioning agent, it is preferred that a cationic polymer also be present.

### Styling polymers

If the product is a styling product it is preferred if a styling polymer is present

The hair styling polymer if present is preferably present in the compositions of the invention in an amount of from 0.001% to 10% by weight, more preferably from 0.1% to 10% by weight, such as from 1% to 8% by weight.

Hair styling polymers are well known. Suitable hair styling polymers include commercially available polymers that contain moieties that render the polymers cationic, anionic, amphoteric or nonionic in nature. Suitable hair styling polymers include, for example, block and graft copolymers. The polymers may be synthetic or naturally derived.

### Adjuvants

The compositions of the present invention may also contain adjuvants suitable for hair care. Generally such ingredients are included individually at a level of up to 2, preferably up to 1 wt% of the total composition.

Suitable hair care adjuvants, include amino acids and ceramides.

The invention will now be further illustrated by the following, non-limiting Examples.

A number illustrates examples of the invention; a letter illustrates Comparative Examples.
All percentages quoted are by weight based on total weight unless otherwise stated.

### EXAMPLES

The following Examples were prepared:

**Table 1**

| Shampoo | | | |
|---|---|---|---|
| | Wt % | | |
| Ingredients | Example A | Example 1 | Example 2 |
| Sodium laurylether sulphate (2EO) | 12.0 | 12.0 | 12.0 |
| Cocoyl amidopropyldimethyl glycine | 0 | 0 | 0 |
| Silicone emulsion | 2.0 | 2.0 | 2.0 |
| Guar hydroxypropyl trimethylammonium chloride | 0.30 | 0.30 | 0.30 |
| Preservative | 0.35 | 0.35 | 0.35 |
| Perfume | 0.42 | 0.42 | 0.42 |
| Citric acid | 0.17 | 0.17 | 0.17 |
| Trimethyl glycine | 1.20 | - | - |
| Trehalose | - | 1.0 | 0.4 |
| Adipic acid | - | - | 0.10 |
| Succinic acid | - | 0.50 | - |
| Guanadine carbonate | - | - | 0.10 |
| Water and minors | to 100 wt% | | |

Results were obtained from a panel of sixty consumers. Each product was tested sequentially for a period of four days, with three days in-between, using a product without any actives. The results show consumer assessed scores of the Examples relative to the Comparative Example.

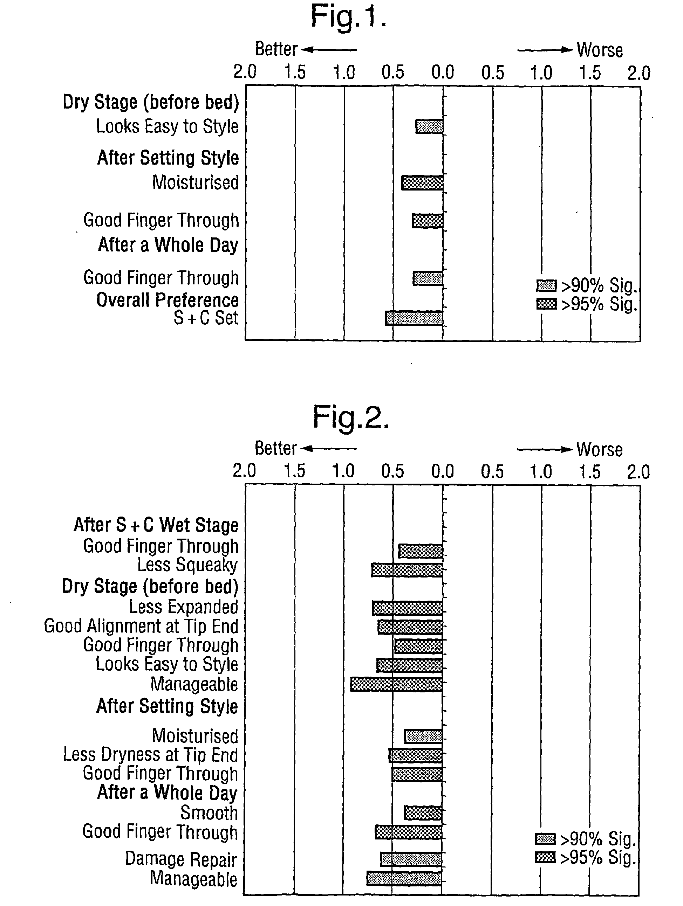

The results are shown in figures 1 and 2. It is clearly shown that both Examples 1 and 2 have significant advantages over the comparative Example (Example A)

## Claims

1. A method of treating hair comprising the step of applying to the hair a composition comprising i) from 0.2 wt.% to 3 wt.% of a disaccharide selected from trehalose, cellobiose or mixtures thereof; and ii) a di-acid of formula:
HOOC-(CH₂)ₙ-COOH
where n is an integer from 2 to 8.

2. A method according to claim 1 in which the disaccharide of the composition is trehalose.

3. A method according to claim 1 where n is equal to 2 or 4.

4. A method according to any preceding claim in which the composition further comprises a guanidinium, salt.

5. A method according to claim 4 in which the guanidinium salt is a carbonate.

6. A method according to any proceeding claim in which the composition further comprises a surfactant.

7. A method according to any preceding claim in which the composition comprises an aqueous base.

8. Use of a composition according to any preceding claim for smoothing hair.

9. Use of a composition according to any one of claims 1 to 7 for aligning hair.

10. Use of a composition according to any one of claims 1 to 7 for preventing damage to the hair.

## Patentansprüche

1. Verfahren zum Behandeln von Haar, umfassend den Schritt des Auftragens auf das Haar einer Zusammensetzung, umfassend i) 0,2 Gewichtsprozent bis 3 Gewichtsprozent von einem Disaccharid, ausgewählt aus Trehalose, Cellobiose oder Gemischen davon; und ii) eine Disäure der Formel:
HOOC-(CH₂)ₙ-COOH,
worin n eine ganze Zahl von 2 bis 8 ist.

2. Verfahren nach Anspruch 1, worin das Disaccharid der Zusammensetzung Trehalose ist.

3. Verfahren nach Anspruch 1, worin n gleich 2 oder 4 ist.

4. Verfahren nach einem vorangehenden Anspruch, worin die Zusammensetzung weiterhin ein Guanidiniumsalz umfasst.

5. Verfahren nach Anspruch 4, worin das Guanidiniumsalz ein Carbonat ist.

6. Verfahren nach einem vorangehenden Anspruch, worin die Zusammensetzung weiterhin ein Tensid umfasst.

7. Verfahren nach einem vorangehenden Anspruch, worin die Zusammensetzung eine wässrige Base umfasst.

8. Verwendung einer Zusammensetzung nach einem vorangehenden Anspruch zum Glätten von Haar.

9. Verwendung einer Zusammensetzung nach einem der Ansprüche 1 bis 7 zum Ausrichten von Haar.

10. Verwendung einer Zusammensetzung nach einem der Ansprüche 1 bis 7 zum Vorbeugen von Schädigung des Haars.

## Revendications

1. Procédé de traitement des cheveux comprenant l'étape consistant à appliquer sur les cheveux une composition comprenant i) de 0,2 % à 3 % en poids d'un disaccharide choisi parmi le tréhalose, le cellobiose ou des mélanges de ceux-ci ; et ii) un diacide de formule :
HOOC-(CH₂)ₙ-COOH
dans laquelle n est un entier compris entre 2 et 8.

2. Procédé selon la revendication 1, dans lequel le disaccharide de la composition est le tréhalose.

3. Procédé selon la revendication 1, dans lequel n est égal à 2 ou à 4.

4. Procédé selon l'une quelconque des revendications précédentes, dans lequel la composition comprend en outre un sel de guanidinium.

5. Procédé selon la revendication 4, dans lequel le sel de guanidinium est un carbonate.

6. Procédé selon l'une quelconque des revendications précédentes, dans lequel la composition comprend en outre un tensioactif.

7. Procédé selon l'une quelconque des revendications précédentes, dans lequel la composition comprend une base aqueuse.

8. Utilisation d'une composition selon l'une quelconque des revendications précédentes pour lisser les cheveux.

9. Utilisation d'une composition selon l'une quelconque des revendications 1 à 7 pour ordonner les cheveux.

10. Utilisation d'une composition selon l'une quelconque des revendications 1 à 7 pour éviter que les cheveux ne s'abîment.
